Europäisches Patentamt

European Patent Office

Office européen des brevets

(11) Publication number: 0 398 677 A2

# EUROPEAN PATENT APPLICATION

(21) Application number: 90305264.5

(22) Date of filing: 16.05.90

(51) Int. Cl.5: C12Q 1/68, C07H 21/04

(30) Priority: 16.05.89 US 352763

(43) Date of publication of application:
22.11.90 Bulletin 90/47

(84) Designated Contracting States:
AT BE CH DE DK ES FR GB IT LI LU NL SE

(71) Applicant: PRESIDENT AND FELLOWS OF
HARVARD UNIVERSITY
1350 Massachusetts Avenue
Cambridge, Massachusetts 02138(US)

(72) Inventor: Wirth, Dyann F.
37 Hancock Street
Boston, Massachusetts 02114(US)
Inventor: Patel, Rubina J.
2445 Lyttonville Road, Apartment 316
Silver Springs, Maryland 20910(US)

(74) Representative: Hutchins, Michael Richard et
al
Graham Watt & Co. London Road Riverhead
Sevenoaks, Kent TN13 2BN(GB)

(54) DNA hybridization probes for identification of mycobacteria.

(57) A nucleic acid sequence specific for genomic nucleic acid of Mycobacterium tuberculosis is disclosed. This sequence is from M. tuberculosis and can hybridize with certain members of the tuberculosis complex of mycobacteria, e.g. M. tuberculosis, M. bovis and the Montreal strain of M. bovis - (Calmette Bacille-Guerin). This invention also pertains to DNA fragments identified by amplification of mycobacterial nucleic acid by the polymerase chain reaction (PCR) technique using oligodeoxyribonucleotide primers derived from this isolated nucleic acid sequence of M. tuberculosis. DNA fragments amplified in this manner will specifically hybridize with nucleic acid from M. tuberculosis.

The invention also pertains to diagnostic methods such as hybridization assays which employ the nucleic acid sequences and to kits for performing the assays.

# DNA HYBRIDIZATION PROBES FOR IDENTIFICATION OF MYCOBACTERIA

## Background

The mycobacteria are a diverse assemblage of acid-fast, gram-positive bacteria, some of which are important disease-causing agents in humans and animals, Bloom, et al., Rev. Infect. Dis., 5:765-780 (1983); Chaparas, CRC Rev. Microbiol., 9:139-197 (1982). In man the two most common mycobacteria-caused diseases are leprosy and tuberculosis, which result from infection with Mycobacterium leprae and Mycobacterium tuberculosis, respectively.

Other mycobacterial species are capable of causing tuberculosis or tuberculosis-like disease. Wallace, R.J., et al., Review of Infectious Diseases, 5:657-679 (1984). Mycobacterium avium, for example, causes tuberculosis in fowl and in other birds. Members of the M. avium-intracellularae complex have become important pathogens among individuals with acquired immunodeficiency syndrome (AIDS). Certain groups of individuals with AIDS have a markedly increased incidence of tuberculosis as well. Pitchenik, A.E., et al., Annals of Internal Medicine, 101:641-645 (1984). Mycobacterium (M.) bovis, is a species which causes tuberculosis in cattle and is transmissible to humans and other animals, in whom it also causes tuberculosis. The "tuberculosis complex" of the family Mycobacteriaceae also includes M. africanum and M. microti. Patel, R.J., et al., Rev. Infect. Diseases, 11: Suppl.2, S411-S419 (1989).

At present, nearly all tuberculosis is caused by respiratory infection with M. tuberculosis. Infection may be asymptomatic in some, but in other individuals, it produces pulmonary lesions which lead to severe debilitation or death.

Today, tuberculosis remains a significant health problem especially in developing countries. Worldwide, an estimated 11 million people are affected with the disease and about 3.5 million new cases occur each year. U.S. Congress, OTA, "Status of Biomedical Research and Related Technology for Tropical Diseases", OTA-H-258, Washington, D.C. 1985.

Diagnostic measures for these mycobacterial diseases are barely adequate. Efficient patient management and control of transmission are compromised by current inadequacies in techniques for the rapid identification of the etiologic agent in the laboratory. Although relatively small numbers of bacilli may be detected by microscopy, Shoemaker, S.A., et al., Am. Res. Respir. Dis., 131:760-763 (1985), Mycobacterium tuberculosis cannot be differentiated from other acid-fast bacilli on the basis of morphology alone. Thus, specimens containing acid-fast microorganisms must be cultured to permit more definitive identification by means of criteria such as growth rate, colonial morphology, biochemical behavior, and drug susceptibility. Vestal, A.L., HEW Publ. No. (CDC)77-8230 Atlanta, 1975; Bates, J.H., Am. Rev. Respir. Dis., 132: 1342 (1985). M. tuberculosis is difficult to culture and has a generation time of 15-20 hours. Wayne, L.G., Am. Rev. Respir. Dis., 125 (Suppl.) 31-41 (1982). A delay of up to 6 weeks before results of laboratory tests are available is not unusual.

In recent years, novel approaches to the identification of mycobacteria have shortened this waiting period to 7-10 days. See, e.g., Gross, W.M., and J.E. Hawkins, J. Clin. Microbiol., 21:565-568, (1985); Knisley, C.V., et. al., J. Clin. Microbiol., 22:761-767, (1985). Most of these methods require that the organisms undergo some replication in vitro. A cDNA probe homologous to ribosomal RNA sequences in M. tuberculosis is available commercially from Gen-Probe (San Diego, CA). Kohne, D.E., WO 8402721 (July 19, 1984); Hogan, J.J., WO 8803957 (June 2, 1988). Diagnostic tests performed using this probe must be performed on cultured organisms because the method has not been tested for direct application to clinical specimens.

There is a need for improved diagnostic assays for M. tuberculosis infection.

## Summary of the Invention

This invention pertains to nucleic acid sequences which hybridize specifically to genomic nucleic acid of Mycobacterium tuberculosis. In particular, this invention pertains to an isolated deoxyribonucleic acid sequence derived from Mycobacterium tuberculosis that hybridizes to nucleic acid from Mycobacterium tuberculosis, Mycobacterium bovis, and the Montreal strain of Mycobacterium bovis (Bacille Calmette-Guerin), but not to nucleic acid from Mycobacterium avium, Mycobacterium intracellulare, and Mycobacterium scrofulaceum. This invention also pertains to nucleic acid sequences identified by amplification of mycobacterial nucleic acid by polymerase chain reaction using oligodeoxyribonucleotide primers derived from the above-described sequence. The nucleic acid sequences of this invention are useful as nucleic acid probes in hybridization assays for the rapid identification of M. tuberculosis.

## Brief Description of the Figures

Figure 1 shows the nucleotide sequence of the mycobacterial insert of pMTb4. Positions of the oligonucleotide primers used in polymerase chain reactions are also shown. Nucleotides denoted by "N" are those that were not determined unambiguously.

Figure 2 shows the nucleotide sequence of two pairs of primers, AB and CD, used in the polymerase chain reactions. The location of these oligonucleotide primers is also shown in Figure 1, supra.

Figure 3 is a Southern blot showing results of a hybridization assay using a radiolabeled PCR-amplified DNA fragment as a probe against selected mycobacteria. The PCR-amplified fragment is derived from the CD primer of Figures 1 and 2 and uses M. tuberculosis DNA as the template.

## Detailed Description of the Invention

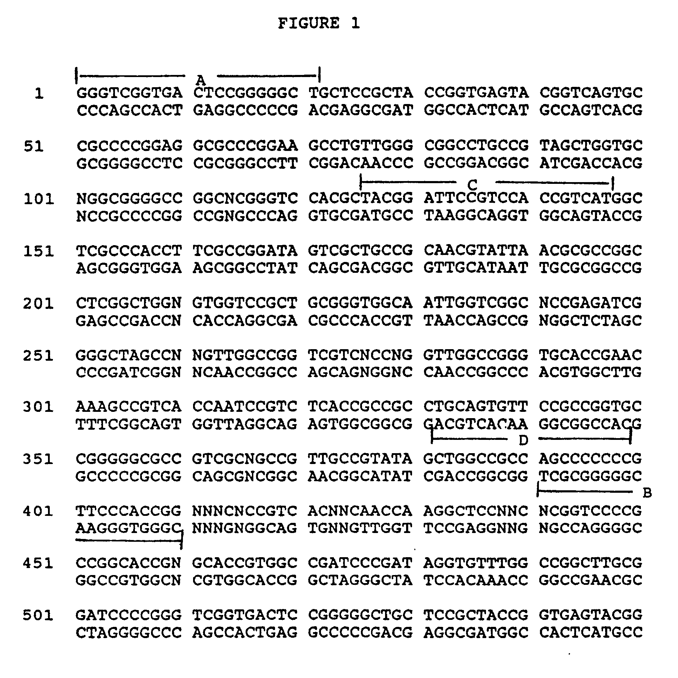

A DNA fragment which specifically hybridizes to nucleic acid of Mycobacterium tuberculosis has been isolated and sequenced. The fragment consists of 1,016 base pairs and its nucleotide sequence is given in Figure 1. The fragment was cloned into plasmid pMTb4, as described by Patel et al., Rev. Infect. Diseases 11, Supplement 2, S411-S419 (1989), the teachings of which are incorporated by reference herein. The cloned fragment consists of two nearly identical fragments of 507 and 509 bases. See Figure 1 and Exemplification. This DNA fragment specifically hybridizes to genomic nucleic acid of M. tuberculosis, M. bovis, and M. bovis BCG (Bacille Calmette-Guerin) Montreal strain. The DNA fragment does not hybridize to M. avium, M. intracellulare, and M. scrofulaceum. This nucleic acid fragment, portions of the fragment, or functionally equivalent variants of the fragment can be used in the hybridization assays for M. tuberculosis, as described below. Functionally equivalent variants include sequences in which base pairs have been deleted, inserted or substituted without substantially affecting the specificity of the sequence for hybridization to M. tuberculosis.

The DNA fragment is also a source of primers for the amplification of mycobacterial DNA sequences by the polymerase chain reaction (PCR) technique. This allows the identification of additional species-specific nucleic acid sequences. Two oligodeoxynucleotides primers, generally about 18-22 nucleotides long, are used that flank a region of the mycobacterial genome (i.e. the template) to be amplified. One primer is complementary to the (+) template strand and the other is complementary to the (-) template strand. One primer is annealed to the (+) strand of denatured genomic template and is extended with DNA-directed DNA polymerase and deoxynucleoside triphosphates. This results in synthesis of a (-) strand fragment which is a replica of the target sequence. Simultaneously, a similar reaction occurs with the other primer, creating a new (+) strand. These newly synthesized DNA strands are themselves templates for the primers. Repeated cycles of denaturation, annealing, and extension result in the amplification of the mycobacterial target sequence(s) defined by the primers. In this manner, DNA sequences are amplified and can be used as DNA probes in hybridization assays. See, e.g. Mullis, U.S. Patent No. 4,683,202, incorporated by reference herein.

Distinct, amplified-DNA fragments can then be identified on chromatographic gels. The specificity of the amplified DNA fragment can be tested by hybridizing the amplified fragment with mycobacterial DNA or by using it as a hybridization probe for PCR products.

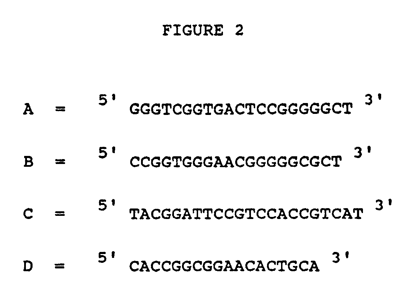

Preferred primer pairs range from 18 to 22 nucleotides in length and are synthesized from the 1016-base pair sequence of pMTb4. These primers are used in polymerase chain reactions with template DNA from mycobacteria. This procedure was carried out with two pairs of oligonucleotide primers (sequences shown in Figure 2). The primers were hybridized to nucleic acid of six ATCC strains of M. tuberculosis and three strains of M. avium. Distinct fragments were amplified and resolved on agarose or polyacrylamide gels stained with ethidium bromide. DNA from these gels were transferred to nitrocellulose filters and were tested for species-specificity with a probe consisting of DNA amplified by polymerase chain reaction with primers derived from M. tuberculosis DNA. As described in more detail below, there was significant probe hybridization with fragments amplified in M. tuberculosis nucleic acid but no detectable hybridization to filters containing M. avium nucleic acid. The PCR-amplified sequence can be used in nucleic acid hybridization assays for the detection of M. tuberculosis.

The methodology described has general applications; it can be used to identify and isolate other species-specific mycobacterial nucleic acid sequences. For example, genomic DNA fragments from a species of mycobacteria can be cloned and sequenced. From the sequence data, oligonucleotide primers can be synthesized for PCR amplification of mycobacterial DNA. The amplified DNA can be tested for species specificity.

Nucleic acid sequences of this invention can be used as probes to detect M. tuberculosis. The hybridization assays can be of various embodiments. In general, a sample of nucleic acid to be tested is incubated with a nucleic acid probe under appropriate conditions of stringency such that it hybridizes specifically to nucleic acid of Mycobacterium tuberculosis. After the incubation, unhybridized probe is removed and the sample is analyzed for hybridized probe as indicative of the presence or the amount of nucleic acid of Mycobacterium tuberculosis.

The nucleic acid to be analyzed can be extracted from mycobacterial cells. Optionally, the extracted DNA can be digested with a restriction enzyme and the resulting DNA fragments separated on the basis of size (e.g., by gel electrophoresis). The DNA can be bound to a nitrocellulose filter. The nitrocellulose-bound fragments are then probed with a labeled nucleic acid probe. The label can be a radioisotope such as $^{32}$P. Autoradiography of the nitrocellulose-bound fragments reveals the occurrence of labeled probe:cell DNA complexes.

In another embodiment, the nucleic acid probes of this invention are used to specifically immobilize or "capture" sample DNA onto a solid support. The probe is bound to a solid support such as polystyrene. The probe can also be bound to a layer or substratum of a support, which is coated directly onto the solid support. The probe can be affixed to the substratum through an oligomeric nucleotide "tail" which can bind to the substratum. Under appropriate conditions the probes hybridize to target nucleic acid sequences in the sample, thus forming a complex consisting of capture probe hybridized to a complementary target DNA sequence. The solid support can be separated from the sample, if necessary, and a generic label added to the solid support. Detection of the label serves as an indicator of mycobacterial DNA from the sample.

The methods for preparing solid supports, substrata, and labeling probes are well known in the art. See, for example Nagata et al., FEBS, 183, 379-382 (1985) describing use of DNA immobilized to polystyrene microtiter wells; Polsky-Cynkin et al., Clinical Chemistry, 31, 1438-1443 (1985), describing the use of immobilized capture probes in clinical assays; Wolf, et al, Nucleic Acids research, 15, 2911-2926 (1987), describing a method for the covalent attachment of oligonucleotides to latex-coated polystyrene beads; Stabinsky, U.S. Patent Number 4,751,177, describing tailed capture probes and solid supports containing oligo-(dT); and Soderland, UK Patent Application GB 2169403A (1985), describing affinity-based capture hybridization methods.

The hybridization assays can be performed on any bacterial sample which is sufficient to allow contact with the probe and for hybridization to occur. The sample is generally pretreated with an agent which disrupts molecular structures within the cells. These agents or procedures will disrupt the cells, present in the sample to release nucleic acids. Such agents are generally compounds or solvents which disrupt the molecular structure of a cell, that is, these agents are capable of denaturing the secondary, tertiary and/or quarternary structures of biopolymers, including proteins, nucleic acids and polysaccharides, that are generally found in specimens. Examples of agents that disrupt molecular structures are chaotropic salts (e.g., guanidinium thiocyanate), and monovalent salts of large acidic anions (e.g. trichloroacetate, trifluoroacetate), denaturing detergents (e.g., dodecyl sulfate), hydrolytic enzymes (e.g., proteases), and compounds which disrupt hydrophobic bonds (e.g., phenols, dimethyl formamide, dimethylsulfoxide, tetramethyl urea, guanidinium hydrochloride) or hydrogen bonds (e.g., urea, formamide). Physical or mechanical means of disrupting molecular structures, e.g., freeze thawing, can also be used to prepare samples for testing. A preferred freeze-thawing technique is to freeze mycobacterial cells to about -70° C and then thaw the sample. This provides a lysate which can be used directly in the hybridization with a nucleic acid probe(s) without the need for separation or filtration steps. Agents that disrupt molecular structures can be used singly or in various combinations to achieve a desired result.

The mycobacterial DNA extracted by these procedures can itself be amplified by the PCR method. Such amplification is desirable if the original sample contains low numbers of target mycobacterial DNA. The DNA primers chosen for this amplification step will depend on which particular target mycobacteria is intended to be detected.

The DNA hybridization probes of this invention can be incorporated into a kit for clinical use. Such a kit would include the nucleic acid probe and, optionally, a means for labeling the probe. The probe can be in free form or it can be bound to a solid support as a capture probe. The kit can also contain solutions or other chaotropic agents for cell lysis, wash solutions and buffers, depending on the particular format of the assay.

The invention is illustrated further by the following examples.

### EXAMPLES

Example 1: Subcloning and sequencing of an M. tuberculosis-specific DNA fragment

This Example illustrates the subcloning and sequencing a 1016-base pair DNA sequence from M. tuberculosis.

## MATERIALS & METHODS

### A. Subcloning for sequencing.

Cloning vectors pBluescript KS (+) and SK (+) (Stratagene Cloning Systems, LaJolla, CA) were digested with HindIII and SalI (New England Biolabs, Beverly, MA) and treated with calf intestine phosphatase (Boehringer Mannheim Biochemicals, Indianapolis, IN). A pBR322 plasmid containing a 460bp fragment of M. tuberculosis was used as the source material. Patel et al., Rev. Infect. Diseases, Supplement 2, S411-S419, (1989) incorporated by reference herein. Ten micrograms of this plasmid was digested sequentially with HindIII, SalI and PvuII and the HindIII-SalI fragment of approximately 1.6 kilobase pairs (kb) containing the mycobacterial insert was force cloned into the pBluescript vectors during overnight incubation with T4 ligase. An aliquot of the ligation reaction was used to transform Escherichia coli JM109. Approximately six white colonies from LB agar plates containing 100 ug/ml ampicillin, 0.33 mM isopropyl 8-D-thiogalactopyranoside (IPTG, from Sigma Chemical Co., St. Louis, MO) and 0.033% 5-bromo-4-chloro-3-indolyl-Beta-D-galactopyranoside (X-gal, from Sigma) were randomly picked for each vector and grown overnight in 5 ml LB broth. Electrophoresis of mini-preparations through a 1% agarose gel showed that all colonies had inserts of the same size. Plasmids pMTB4KS2 and pMTb4SK2 were purified by cesium chloride density centrifugation.

### B. Nested deletions for sequencing

The plasmid PMTb4SK2 was used as a prototpe to make nested deletions. Plasmid pMTb4SK2 was digested sequentially with KpnI and SalI and purified with phenol/chloroform and ethanol precipitated. Linearized DNA was digested with Exonuclease III for 0, 2, 4, 6, 8 and 10 minutes at room temperature. At each time point an aliquot of the digest was transferred to a tube containing Mung Bean Nuclease maintained on ice. These tubes were then incubated together at 30°C for 30 minutes. After phenol/chloroform treatment and ethanol precipitation, the blunt-ended DNA was incubated overnight with T4 ligase. Escherichia coli JM109 were transformed with the circularized molecules and plated on LB agar containing ampicillin, IPTG, and X-gal. Stock cultures of 26 white colonies were made from 5 ml LB broth cultures and stored at -20°C. Clones containing the appropriate deletions were identified from mini-preparations run on agarose gels.

### C. Preparation of DNA and sequencing by dideoxynucleotide chain termination

Single-stranded DNA (ssDNA) was obtained by co-culturing transformed E. coli with helper phages R408 or VCS-M13 in 5 or 50 ml of medium. Protocols were followed or modified slightly to sequence ssDNA from pMTb4SK 4-1, pMTb4SK4-2 and pMTb4SK4-3 using the Klenow fragment of DNA polymerase (New England Biolabs) or the modified bacteriophage T7 DNA polymerase, Sequenase (United States Biochemicals, Cleveland, OH). For every set of reactions, commercially prepared ssDNA from bacteriophage M13 was included as control. Double-stranded sequencing (Internatonal Biotechnologies, Inc., New Haven, CT) was performed on pMTb4KS2.

All sequencing reactions were done using $^{35}$S-labelled dATP. Reactions were stored at -80°C or maintained on ice prior to loading on denaturing 6% polyacrylamide gels, and depending upon the protocol, heated to 70-95°C for two minutes immediately before loading. Modifications in the vendors' procedures included: Klenow reactions done at 50°C; Sequence reactions done at 50°C; shorter reaction times with Sequenase; the use of Taq polymerase (Perkin Elmer Cetus, Norwalk, CT) at 72°C with the Sequenase kit; and loading samples directly after heating at 100°C.

An alternative to making nested deletions was to synthesize olignucleotide primers and sequence in a unidirectional fashion. The pBR322 sequence was obtained from Maniatis et al. Molecular Cloning: A Laboratory Manual, Cold Spring Harbor Laboratory, 1982, or a computer program "DNA Inspector II+" (Textco, W. Lebanon, NH). Primers were generated when required, starting from approximately 40 bases away from the destroyed pBR322 EcoRV sites. In pMTb4SK2 five primers were generated to determine the sequence of more than 1,106 nucleotides. This M. tuberculosis-specific sequence is comprised of two almost identical fragments of 507 and 509 bases with a difference of 14 nucleotides (Figure 1).

### D. Polyacrylamide gel electrophoresis and autoradiography.

Aliquots of sequencing reactions were loaded on 6% polyacrylamide gels containing either 7M urea and a buffer gradient of 0.5X to 1.5X Tris-borate-EDTA (TBE), or 8M urea and 1X TBE. Gels were pre-run at 100 Watts to bring the temperature of the gel and buffer chamber (Bio-Rad Laboratories, Rockville Center, NY) up to 55˚C. Samples were loaded with a shark's tooth comb and run until the bromophenol blue reached the bottom of the gel, usually two hours for non-gradient and two and a half hours for gradient gels. In some cases another aliquot of the reactions was loaded in adjacent lanes to increase the number of readable bases per gel.

Gels were immersed in 10% MeOH, 10% glacial acetic acid for 45 to 60 minutes, transferred to blotting paper (Schleicher & Schuell, Keene, NH) and dried for 45 to 60 minutes on a gel-dryer (Bio-Rad) set at 80˚C. The gel was exposed to Kodak X-Omat AR film at -80˚C for 24 to 48 hours.


RESULTS


Recombinant Bluescript Plasmids contain the HindIII - SalI fragment from pmTb4 in each orientation were designated pMTb4KS2 and pMTb4SK2. E. coli - JM109, when transformed with these plasmids, replicated much more slowly than when transformed with recombinants of pBR322 or pUC vectors. When purified over cesium chloride, the yield of pMTb4SK2 DNA (5.136 mg per 500 ml culture) was better than that of pMTb4SK2 (0.949 mg per 500 ml culture). Longer incubation was required for colonies to turn blue on IPTG/X-gal agar plates, and digestion with restriction endonucleases was incomplete even at high enzyme concentrations.

pMTb4SK2 was used as a prototype to make nested deletions. Of the twenty-six transformants obtained after treatment with Exonuclease III, Mung Bean Nuclease, and T4 ligase, three clones (pMTb4SK4-1, pMTb4SK4-2 and pMTb4SK4-3) from the four-minute time point seemed to contain the appropriate deletions. At first, ssDNA was obtained from these three clones by co-culturing with helper phage R408 in 5 ml of medium. Ultimately, helper phage VCS-M13 was used to rescue ssDNA from pMTb4KS2 and pMTb4SK2 in 50 ml cultures. pMTb4KS2 proved to be troublesome. Very small (and dilute) quantities of ssDNA were recovered, even from 50 ml cultures. Furthermore, the helper phages seemed to be amplified rather than the single-stranded pMTb2KS2.

Initially, sequencing reactions were done with Klenow polymerase at 37˚C, heated to 70˚C and loaded on 6% buffer gradient polyacrylamide gels to maximize the number of readable bases. Of the three clones identified, pMTb4SK4-2 provided the best sequence data of approximately 300 nucleotides. The sequence obtained from pMTb4SK4-3 was that of a region close to the HindIII site in pBR322. No sequence was obtained from pMTb4SK4-1. Attempts at double-stranded sequencing using pMTb5 (Patel et al., supra) which contained part of the mycobacterial sequence present in pMTb4, and with pMTb4KS2, met with little success.

By synthesizing primers when necessary, it was possible to "walk" in a unidirection fashion along the mycobacterial fragment in pMT4bSK2. This is a more efficient method than making deletions and trying to identify clones containing the deletions. Primers were generated when required starting from about 40 base pairs away from the destroyed pBR322 EcoRV sites. Under the best of conditions, only the first 125 bases could be determined in pMTb4KS2. In pMTb4SK2, however, five primers were generated and used to determine the pMTb4SK2 sequence. This allows for the correction of a BamH1 site in the previously constructed map of pMTb4 which had been determined by restriction endonoclease digestion. Patel et al., supra, Figure 2, page S416.

The mycobacterial insert in pMTb4 is comprised of two nearly identical fragments of 507 and 509 base pairs (Figure 1). This Figure shows only the first of the two nearly identical dimeric fragments. The nucleotide sequence was analyzed using computer programs "PCGENE" and DNA Inspector II+ (Intelligenetics, Inc.). Combining the results, the guanine plus cytosine (GC) content was 69% which is consistent with published reports. A data bank (Intelligenetics) was screened to find any possible homologies, especially with insertion sequences. No significant homology was found.


EXAMPLE 2


Amplification with polymerase chain reaction (PCR) and Southern hybridization with a M. tuberculosis specific probe


MATERIALS AND METHODS


A. Synthesis of oligonucleotide primers

Primers derived from the nucleotide sequence determined from pMTb4SK2 and ranging from 17 to 22 nucleotides were generated on an automated DNA synthesizer (BIOSEARCH 8600, San Rafael, CA) based on phosphoamidite chemistry. After synthesis, the primer solution was transferred to glass vials and incubated with concentrated ammonium hydroxide at 50°C for 4 hours. The ammonium hydroxide was evaporated in a Speed-Vac/Concentrator (Savant Instruments, Inc., Farmingdale, NY) and the pellet was washed and dried twice with 0.5ml sterile, deionized, distilled water. The pellet was finally dissolved in 0.5 ml 10 mM Tris-HCl, ph 8.0, 1 mM EDTA (TE). The concentration of each primer was determined spectrophotometrically and an aliquot was loaded on a polyacrlamide gel to verify that it was not degraded.

B. Amplification with polymerase chain reaction (PCR)

Reaction buffer for PCR's consisted of 50 mM KCl, 10 mM Tris-HCl, pH 8.4, 1.5 mM MgCl₂, and 0.01% gelatin. The 100 ul reaction mixture contained DNA, primers to 1 uM, 1X reaction buffer, and dATP, dCTP, dGTP, and dTTP to 0.2 mM primers. The volume was made up with sterile, deionized, distilled water. The reaction mixture was incubated at 95°C for the first denaturation step, tubes were contrifuged momentarily, and immediately put on ice while Taq polymerase and mineral oil were added. Annealing temperature was either 37°C or 55°C and synthesis was carried out at 72°C. The reactions were processed either manually or by a DNA thermal cycler (Perkin Elmer Cetus). At the end of 25 cycles the tubes were allowed to cool to room temperature, centrifuged and then as much of the mineral oil was removed as possible. Reactions were stored at 4°C.

One-tenth volumes (10 ul) of the reactions were loaded on either 4% NuSieve (FMC Bioproducts, Rockland, ME), 3% NuSieve plus 1% agarose, 1.8% agarose, or 6% polyacrylamide gels and run in 1X TBE buffer for two hours at 100V (Patel et al., 1989, supra). The gels were stained in 0.5 ug/ml ethidium bromide and PCR products visualized by ultraviolet light were photographed with Polaroid 667 film as previously described (Patel et al., 1989, supra). Standard procedures (Maniatis et al., supra) were followed for Southern transfers, nick translations and autoradiography or modified as described previously (Patel et al., 1989, supra).

RESULTS

Using the nucleotide sequence determined from pMTb4SK2 (Figure 1), two pairs of primers, AB and CD, were synthesized as shown in Figure 2. These primers were used in polymerase chain reactions with DNA from M. tuberculosis and M.

avium. Using 1 ug of DNA, many fragments were amplified in M. tuberculosis DNA with primers AB and to a lesser extent with CD. Strains of M. tuberculosis used herein were: M. tuberculosis H37RV ATCC 23618 and ATCC 25618; M. tuberculosis H37Ra ATCC 25177; M. tuberculosis H4Ra ATCC 35817; M. tuberculosis Erdman ATCC 35801; M. tuberculosis Indian ATCC 35811 and M. tuberculosis Aoyama ATCC 35813.

Of interest is that there is some amplification in M. avium DNA with primers AB but nothing was readily visualised with primers CD. M. avium DNA used in these procedures was extracted from M. avium strains ATCC 25291, M. avium Pasteur 8063, and an unidentified M. avium isolated from soil.

The DNA in these gels was transferred to nitrocellulose filters which were then probed with [alpha-32P]-labelled PCR product. There was hybridization with fragments amplified in M. tuberculosis DNA but no detectable hybridization to filters containing M. avium DNA. When 1:10 dilutions ranging from 1 ng to 1 fg of M. tuberculosis DNA were used in a PCR with primers AB, a distinct band could be seen in an ethidium bromide stained gel in the 1 ng lane.

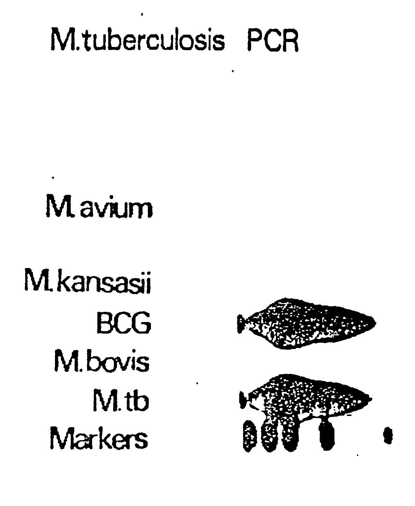

Figure 3 shows results of a Southern blot in which primer CD was used to amplify DNA of M. tuberculosis. The major PCR-amplified band was cut out of the gel and used as a radiolabelled hybridization probe against various mycobacterial DNA shown in Figure 3. The PCR-amplified "CD probe" hybridized most strongly to M. tuberculosis. It also hybridized to M. bovis BCG, but not to M. avium and M. kansasii.

Plasmid Deposit

Plasmid pMTb4 has been deposited with the American Type Culture Collection on March 20, 1986 in E. coli K12 HB101, ATCC No. 67045.

Equivalents

Those skilled in the art will recognize, or be able to ascertain using no more than routine experimentation, many equivalents to the specific embodiments of the invention described specifically herein. Such equivalents are intended to be encompassed in the scope of the following claims.

Claims

1. An isolated nucleic acid sequence which hybridizes specifically to genomic nucleic acid of Mycobacterium tuberculosis.

2. An isolated deoxyribonucleic acid sequence of Mycobacterium tuberculosis that hybridizes to nucleic acid from Mycobacterium tuberculosis, Mycobacterium bovis, and Montreal strain of Mycobacterium bovis (Bacille Calmette-Guerin), but not to nucleic acid from Mycobacterium avium, Mycobacterium intracellulare, and Mycobacterium scrofulaceum.

3. A nucleic acid sequence of Claim 2 having the nucleotide sequence of Figure 1 or the complement thereof.

4. An isolated deoxyribonucleic acid sequence identified by amplification of mycobacterial nucleic acid with the polymerase chain reaction, the polymerase chain reaction utilizing a pair of oligodeoxyribonucleotide primers derived from the nucleic acid sequence of Claim 1.

5. An isolated deoxyribonucleic acid sequence of Claim 4, wherein the pair of oligodeoxyribonucleotide primers have the nucleotide sequences shown in Figure 2.

6. A cloning vector containing the nucleic acid of Claim 1.

7. The plasmid pMTb4, ATCC accession number 67045.

8. A method of detecting nucleic acid of Mycobacterium tuberculosis, comprising contacting a sample of nucleic acid with a nucleic acid probe which hybridizes specifically to nucleic acid of Mycobacterium tuberculosis; incubating the probe and the sample under conditions which permit the labeled probe to hybridize specifically with complementary nucleic acid sequences; removing unhybridized probe; and analysing the sample for hybridized probe as indicative of the presence of nucleic acid of Mycobacterium tuberculosis.

9. A method of Claim 8, wherein the nucleic acid probe hybridizes to nucleic acid from Mycobacterium tuberculosis, Mycobacterium bovis, and the Montreal strain of Mycobacterium bovis - (Bacille Calmette-Guerin), but not to nucleic acid from Mycobacterium avium, Mycobacterium intracellulare, and Mycobacterium scrofulaceum.

10. A method of Claim 9, wherein the nucleic acid probe has the nucleotide sequence of Figure 1 or the complement thereof.

11. A method of Claim 10, wherein the probe comprises a deoxyribonucleic acid sequence identified by amplification of mycobacterial nucleic acid by the polymerase chain reaction with a pair of oligodeoxynucleotide primers derived from the nucleic acid sequence of Claim 1.

12. A method of Claim 11, wherein the pair of oligodeoxynucleotide primers have the nucleotide sequences shown in figure 2.

13. A method of Claim 8, wherein the nucleic acid probe is labeled, for example with a radioisotope.

14. A method of Claim 8, wherein the nucleic acid to be tested is amplified by the polymerase chain reaction technique.

15. A method of Claim 8, wherein the sample is a cell lysate prepared by freeze-thawing mycobacterial cells, for example, wherein the mycobacterial cells are frozen to about -70°C, then thawed.

# FIGURE 1

```
        |———————— A ————————|
1    GGGTCGGTGA CTCCGGGGGC TGCTCCGCTA CCGGTGAGTA CGGTCAGTGC
     CCCAGCCACT GAGGCCCCCG ACGAGGCGAT GGCCACTCAT GCCAGTCACG

51   CGCCCCGGAG GCGCCCGGAA GCCTGTTGGG CGGCCTGCCG TAGCTGGTGC
     GCGGGGCCTC CGCGGGCCTT CGGACAACCC GCCGGACGGC ATCGACCACG
                                 |————————— C ——————————|
101  NGGCGGGGCC GGCNCGGGTC CACGCTACGG ATTCCGTCCA CCGTCATGGC
     NCCGCCCCGG CCGNGCCCAG GTGCGATGCC TAAGGCAGGT GGCAGTACCG

151  TCGCCCACCT TCGCCGGATA GTCGCTGCCG CAACGTATTA ACGCGCCGGC
     AGCGGGTGGA AGCGGCCTAT CAGCGACGGC GTTGCATAAT TGCGCGGCCG

201  CTCGGCTGGN GTGGTCCGCT GCGGGTGGCA ATTGGTCGGC NCCGAGATCG
     GAGCCGACCN CACCAGGCGA CGCCCACCGT TAACCAGCCG NGGCTCTAGC

251  GGGCTAGCCN NGTTGGCCGG TCGTCNCCNG GTTGGCCGGG TGCACCGAAC
     CCCGATCGGN NCAACCGGCC AGCAGNGGNC CAACCGGCCC ACGTGGCTTG

301  AAAGCCGTCA CCAATCCGTC TCACCGCCGC CTGCAGTGTT CCGCCGGTGC
     TTTCGGCAGT GGTTAGGCAG AGTGGCGGCG GACGTCACAA GGCGGCCACG
                                         |————————— D ——————————|
351  CGGGGGCGCC GTCGCNGCCG TTGCCGTATA GCTGGCCGCC AGCCCCCCCG
     GCCCCCGCGG CAGCGNCGGC AACGGCATAT CGACCGGCGG TCGCGGGGGC
                                                 |———————————— B
401  TTCCCACCGG NNNCNCCGTC ACNNCAACCA AGGCTCCNNC NCGGTCCCCG
     AAGGGTGGGC NNNGNGGCAG TGNNGTTGGT TCCGAGGNNG NGCCAGGGGC
     ————————————————|
451  CCGGCACCGN GCACCGTGGC CGATCCCGAT AGGTGTTTGG CCGGCTTGCG
     GGCCGTGGCN CGTGGCACCG GCTAGGGCTA TCCACAAACC GGCCGAACGC

501  GATCCCCGGG TCGGTGACTC CGGGGGCTGC TCCGCTACCG GTGAGTACGG
     CTAGGGGCCC AGCCACTGAG GCCCCCGACG AGGCGATGGC CACTCATGCC
```

FIGURE 2

A = 5' GGGTCGGTGACTCCGGGGGCT 3'

B = 5' CCGGTGGGAACGGGGGCGCT 3'

C = 5' TACGGATTCCGTCCACCGTCAT 3'

D = 5' CACCGGCGGAACACTGCA 3'

M.tuberculosis  PCR

M. avium

M. kansasii

BCG

M. bovis

M. tb

Markers